(19) Europäisches Patentamt
European Patent Office
Office européen des brevets

(11) EP 0 561 928 B1

(12) **FASCICULE DE BREVET EUROPEEN**

(45) Date de publication et mention de la délivrance du brevet:
**28.02.1996 Bulletin 1996/09**

(21) Numéro de dépôt: **92901021.3**

(22) Date de dépôt: **05.12.1991**

(51) Int Cl.[6]: **C07D 307/46**

(86) Numéro de dépôt international:
**PCT/FR91/00971**

(87) Numéro de publication internationale:
**WO 92/10486 (25.06.1992 Gazette 1992/14)**

# (54) PROCEDE DE PREPARATION D'HYDROXYMETHYL-5 FURFURAL PAR CATALYSE HETEROGENE

VERFAHREN ZUR HERSTELLUNG VON HYDROXYMETHYL-5-FURFURAL DURCH HETEROGENE KATALYSE

METHOD FOR PREPARING 5-HYDROXYMETHYL FURFURAL BY CONTACT CATALYSIS

(84) Etats contractants désignés:
**BE CH DE DK ES GB IT LI NL**

(30) Priorité: **07.12.1990 FR 9015350**

(43) Date de publication de la demande:
**29.09.1993 Bulletin 1993/39**

(73) Titulaires:
- **COMMISSARIAT A L'ENERGIE ATOMIQUE**
  **F-75015 Paris (FR)**
- **FURCHIM**
  **F-97231 Robert (FR)**
- **AGRICHIMIE**
  **F-97231 Robert (FR)**

(72) Inventeurs:
- **AVIGNON, Gérard**
  **F-47310 Estillac (FR)**
- **DURAND, Robert**
  **F-34830 Clapiers (FR)**
- **FAUGERAS, Pierre**
  **F-30310 Pont-Saint-Esprit (FR)**
- **GENESTE, Patrick**
  **F-34000 Montpellier (FR)**
- **MOREAU, Claude**
  **F-34880 Laverune (FR)**
- **RIVALIER, Patrick**
  **F-34130 Mauguio (FR)**
- **ROS, Pierre**
  **F-30150 Sauveterre (FR)**

(74) Mandataire: **Des Termes, Monique et al**
**Société Brevatome**
**25, rue de Ponthieu**
**F-75008 Paris (FR)**

(56) Documents cités:
**FR-A- 2 551 754** **GB-A- 2 058 070**

- **INFORMATIONS CHEMIE no. 212, 1981, pages 179 - 184; A. GASET ET AL.: 'Procédés d'obtention de l'hydroxyméthyl-5-furannecarboxaldéhyde-2'**
- **STÄRKE vol. 42, no. 8, 1980, WEINHEIM (DE) pages 314 - 321; B. F. M. KUSTER:**
- **'5-Hydroxymethylfurfural (HMF). A review focussing on its manufacture'**
- **Biomass, n 14, 1987, pp. 185-194**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

EP 0 561 928 B1

Printed by Jouve, 75001 PARIS (FR)

**Description**

La présente invention a pour objet un procédé de préparation d'hydroxyméthyl-5 furfural (HMF) de formule :

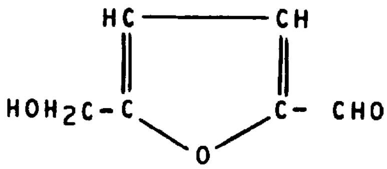

par catalyse hétérogène à partir d'une solution comprenant au moins un hexose ou un oside qui par hydrolyse donne au moins un hexose.

Le HMF, du fait de l'existence dans sa structure de doubles liaisons conjuguées et de fonctions éther, alcool primaire et aldéhyde, présente une grande réactivité, et constitue de ce fait une molécule intéressante pour la synthèse chimique de composés intermédiaires tels que des dialdéhydes, dialcools, diacides et aminoalcools, pour la production de matières plastiques de hautes performances, d'agents tensioactifs, de produits phytosanitaires, etc.

Le HMF peut être obtenu à partir des hexoses par une réaction de déshydratation suivie d'une cyclisation en présence d'un catalyseur acide, ce qui correspond au schéma réactionnel suivant :

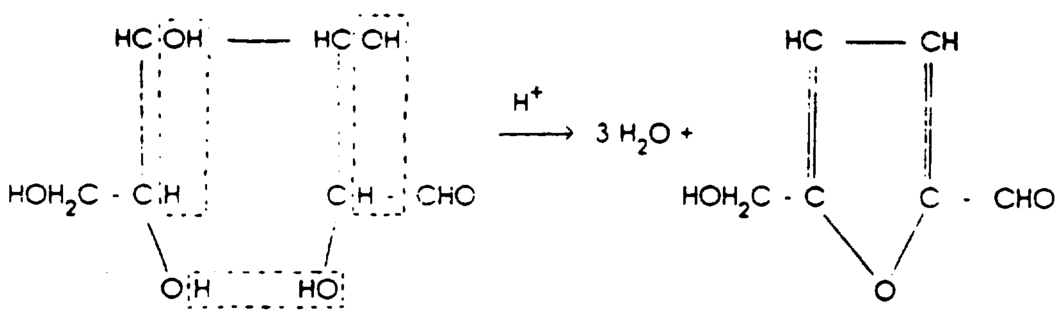

Le mécanisme de formation du HMF à partir des hexoses est cependant plus complexe car il fait intervenir des produits intermédiaires mal définis qui se transforment en HMF mais qui sont susceptibles également de se transformer en polymères insolubles. De même, le HMF, dans les conditions de la réaction, peut être converti par un processus acido-catalysé en acide lévulinique et en acice formique ainsi que se polymériser.

Les réactions produites lors de la déshydratation des hexoses en milieu acide peuvent donc être schématisées sous la forme suivante :

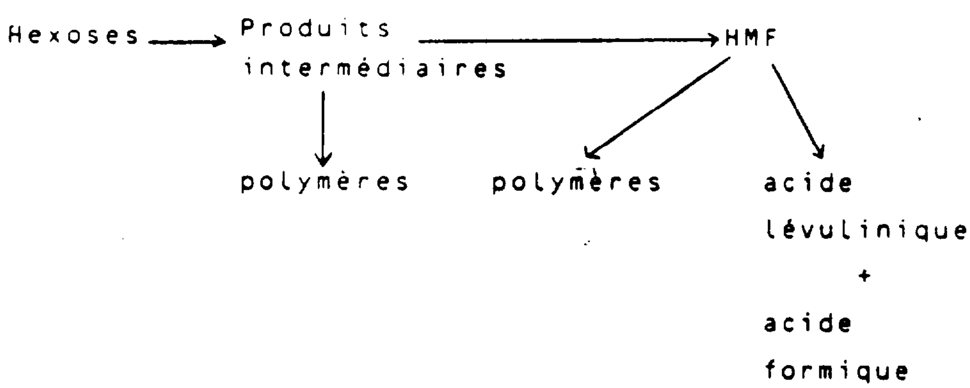


Ainsi, le document Biomass, n°14, 1987, pp 185-194, décrit la transformation de D-fructose en acide lévulinique par déshydratation catalytique du D-fructose fondu sur un catalyseur constitué par la zéolite LZY.

Jusqu'à présent, la transformation des hexoses. en HMF a été réalisée en phase homogène au moyen de catalyseurs acides constitués par des acides minéraux ou organiques tels que les acides chlorhydrique, phosphorique, sulfurique et oxalique, ce qui conduit à certains problèmes de corrosion des installations, de récupération des catalyseurs acides, et surtout d'instabilité du HMF dans ces milieux acides. Aussi, on a envisagé de remplacer cette catalyse en phase homogène par une catalyse en phase hétérogène en utilisant des supports catalytiques solides tels qu'une résine

échangeuse à fonction cationique, comme il est décrit dans le document FR-A-2 464 260.

Avec cette technique de catalyse en phase hétérogène, on peut récupérer le HMF dans un solvant organique, ce qui permet de protéger le HMF des effets néfastes de la température et de l'acidité en l'extrayant du milieu au fur et à mesure de sa formation.

Cependant, les catalyseurs solides utilisés dans ce document, qui sont des résines échangeuses de cations comportant des groupes acides, en particulier des groupes sulfoniques, ne peuvent travailler au-dessus de 100°C, sont difficiles à régénérer et conduisent à un coût de production du HMF élevé, ce qui pose des problèmes pour une production rentable à l'échelle industrielle.

La présente invention a précisément pour objet un procédé de préparation de l'hydroxyméthyl-5 furfural par catalyse hétérogène, qui permet d'obtenir le HMF avec un rendement et une pureté suffisamment élevés pour envisager sa production industrielle à un coût de revient raisonnable.

Selon l'invention, le procédé de préparation d'hydroxyméthyl-5 furfural par mise en contact d'un milieu contenant au moins un hexose ou un oside qui par hydrolyse donne au moins un hexose, avec un catalyseur solide acide, à une température supérieure à 50°C, se caractérise en ce que le catalyseur acide est un tectosilicate ou une argile non fonctionnalisé par des groupes acides tels que COOH et $SO_3H$, sous forme protonique, et en ce que le milieu contenant l'hexose ou l'oside est un milieu aqueux.

Les tectosilicates sont des composés minéraux formés par des enchaînements tridimensionnels de tétraèdres $TO_4$ avec T représentant un élément de la classification périodique des éléments tels que Si, Al, B, Ga, Ge, P, etc., les tétraèdres $TO_4$ de l'enchaînement comprenant au moins deux éléments différents. Dans cet enchaînement, les atomes d'oxygène des tétraèdres $TO_4$ sont en commun avec les tétraèdres voisins.

A titre d'exemple, ces tectosilicates peuvent être des aluminosilicates, des gallosilicates, des aluminogermanates ou des gallogermanates.

Ces tectosilicates sont des composés micro-poreux caractérisés par une structure comportant :

- une charpente tridimensionnelle formée par l'enchaînement des tétraèdres $TO_4$, et
- des canaux et cavités de dimensions moléculaires contenant des cations de compensation éventuels, de l'eau ou d'autres molécules ou sels.

Dans l'invention, on utilise des tectosilicates sous forme protonique ; dans ce cas, les cations de compensation sont constitués par des protons $H^+$ conférant un caractère acide au tectosilicate.

De même, on utilise des argiles sous forme protonique.

On peut utiliser en particulier des zéolithes et des argiles sous forme $H^+$, par exemple une mordénite, une faujasite, une zéolithe β, une zéolithe Y ou une montmorillonite.

Les zéolithes ont par exemple la composition suivante :

$$x\,M^{+}_{1/n}(y_1T^1O_2,\ y_2T^2O_2...)_xZA$$

dans laquelle M est un cation de valence n, $T^1$ et $T_2$ sont des éléments de la charpente, A est une molécule présente dans les canaux, par exemple de l'eau et x, $y_1$, $y_2$ et Z sont des nombres supérieurs à 0.

Dans l'invention, $M^+$ représente de préférence $H^+$.

Une zéolithe utilisable est la zéolithe Y de formule $|Na_{56}\ (AlO_2)_{56}\ (SiO_2)_{136}|\ 264H_2O$ lorsqu'elle est sous forme sodium.

Pour obtenir la zéolithe sous forme protonique, on peut par exemple soumettre la zéolithe sous forme Na à un échange dans une solution aqueuse de chlorure d'ammonium puis la calciner entre 300°C et 600°C pour obtenir la forme protonique.

Avec ces zéolithes, on peut contrôler l'activité protonique par le rapport Si/Al.

Avantageusement, on utilise une zéolithe ayant un rapport Si/Al allant de 2 à 100 en fonction du rapport acidité/hydrophobicité recherché.

Selon l'invention, on utilise les propriétés particulières des tectosilicates et des argiles sous forme protonique pour catalyser la réaction de conversion de l'hexose ou de l'oside en HMF.

Ainsi, on utilise tout d'abord les propriétés acides du tectosilicate (acidité de Lewis ou de Bronsted) pour transformer l'hexose ou l'oside en HMF. Dans le procédé de l'invention, le tectosilicate sert de catalyseur et non de support d'une phase active jouant le rôle de catalyseur comme c'est le cas dans FR-A-2 464 260 où la silice-alumine joue le rôle de support et les groupes sulfoniques constituent la phase active catalytique.

Par ailleurs, dans l'invention, on utilise les tectosilicates comme tamis moléculaire pour piéger les produits secondaires de la réaction. En effet, la microporosité des tectosilicates permet de réaliser, en contrôlant les dimensions des canaux, une sélection des molécules par leur taille.

Ainsi, en utilisant un tectosilicate capable d'adsorber, à l'intérieur de son réseau microporeux, les produits secondaires néfastes formés lors de la réaction, c'est-à-dire l'acide lévulinique et l'acide formique, sans adsorber le HMF qui

ne peut entrer à l'intérieur des micropores, on peut obtenir une meilleure pureté du HMF formé et éviter les réactions secondaires acido catalysées de dégradation du HMF formé.

A titre d'exemple de tectosilicates ayant cette sélectivité d'adsorption, on peut citer les mordénites.

De plus, les tectosilicates et les argiles sous forme protonique utilisés dans l'invention ont l'avantage de supporter des températures beaucoup plus élevées, par exemple 600°C que celles (au plus 100°C) auxquelles résistent les résines fonctionnalisées de FR-A-2 464 260.

Ainsi, on peut accélérer la réaction de synthèse du HMF en opérant au-dessus de 100°C, ce qui était impossible avec les catalyseurs solides fonctionnalisés de FR-A-2 464 260.

Dans le procédé de l'invention, le milieu liquide est un milieu aqueux, mais l'on peut aussi utiliser comme milieu réactionnel un système binaire constitué d'eau et d'un solvant organique non miscible à l'eau.

Dans ce cas, on choisit de préférence le solvant organique de façon à extraire dans celui-ci l'hydroxyméthyl-5 furfural formé.

Dans le procédé de l'invention, grâce au choix d'un catalyseur acide du type tectosilicate et d'un milieu aqueux, le HMF formé se trouve dilué dans l'eau à un pH plus élevé que celui nécesaire pour sa formation. On obtient ainsi une phase aqueuse peu acide qui est moins propice à la dégradation du HMF formé, ce qui permet d'éviter l'apparition de produits secondaires de polymérisation du HMF ainsi que la formation d'acide lévulinique et d'acide formique.

Par ailleurs, étant donné que les tectosilicates utilisés dans l'invention sont facilement régénérables et réutilisables, on évite l'emploi de réactifs de régénération polluants et on peut faire des économies sur le traitement des effluents. De plus, ces catalyseurs solides ne se dégradent pas dans les conditions de réactions utilisées pour la formation du HMF.

Selon l'invention, les conditions réactionnelles utilisées pour la mise en contact de la solution aqueuse contenant au moins un hexose ou un oside constitué par au moins un hexose avec le catalyseur acide sont les conditions classiques de température et de pression.

Dans le procédé de l'invention, on peut réaliser la mise en contact de la solution aqueuse d'hexose avec le catalyseur acide à une température et une pression situées dans la gamme des températures et de pressions utilisées habituellement pour la préparation du HMF.

De préférence, la température est de 100 à 200°C pour avoir une meilleure activité du catalyseur.

De préférence également, on opère sous une pression supérieure à la pression atmosphérique, par exemple sous une pression de 0,1 à 2 MPa, pour rester en particulier en milieu liquide.

La solution aqueuse de départ peut comprendre un ou plusieurs hexoses choisis parmi les aldohexoses et les cétohexoses.

Parmi les aldohexoses, on peut citer par exemple le glucose, le galactose, le mannose et l'idose. Les cétohexoses sont par exemple le fructose, le lévulose, le sorbose, le tagatose et l'allose.

Les osides sont par exemple le saccharose, l'inuline.

Avantageusement, l'hexose est le fructose ou un mélange glucose-fructose. Dans ce dernier cas, en choisissant de façon appropriée les conditions de réaction, on peut obtenir une spécificité vis-à-vis du fructose et ne pas transformer le glucose, ce qui permet de l'utiliser ensuite pour d'autres synthèses.

Dans le procédé de l'invention, on peut extraire en continu l'hydroxyméthyl-5 furfural formé dans le milieu aqueux au moyen d'un solvant organique approprié.

Dans ce but, on utilise un solvant organique qui soit le moins miscible possible avec l'eau à la température de la réaction et qui soit un bon solvant du HMF produit, soit un solvant sélectif du HMF, très peu soluble dans L'eau, dans lequel les hexoses et l'eau sont pratiquement insolubles et de point d'ébullition inférieur à celui du HMF.

A titre d'exemple de tels solvants, on peut citer les cétones, par exemple la méthylisobutylcétone. On peut aussi utiliser des nitriles, des éthers, des alcools non miscibles à l'eau, des dérivés halogénés comme le chlorobenzène et le dichlorobenzène, des hydrocarbures aliphatiques ou aromatiques et des nitroalcanes.

Pour réaliser cette extraction du HMF dans le solvant, on peut effectuer la réaction en milieu triphasique en ajoutant un solvant organique tel que la méthylisobutylcétone à la phase aqueuse contenant l'hexose et le catalyseur solide en suspension. Dans ce cas, la catalyse a lieu dans la phase aqueuse où se cantonne le catalyseur à condition de choisir un solvant qui ne mouille pas le catalyseur, et le HMF est relargué dans la phase organique dès sa formation. Ainsi, le catalyseur ne se désactive que très peu. Le solvant organique peut circuler à co-courant ou à contre-courant de la phase aqueuse contenant le catalyseur solide en suspension.

On peut aussi effectuer la réaction avec un lit fixe ou mobile de catalyseur et extraire par un solvant organique le HMF formé en phase aqueuse dans une deuxième étape après passage de la phase aqueuse contenant les hexoses sur le catalyseur solide. Dans ce cas, on peut opérer par charges ou en continu en mettant en circulation cette phase aqueuse sur le lit fixe ou mobile du catalyseur, et extraire ensuite le HMF par mise en contact de la phase aqueuse sortant du lit de catalyseur avec un solvant organique. On peut aussi extraire en continu le HMF formé en faisant circuler dans le lit fixe ou mobile, le solvant organique à co-courant ou à contre-courant de la phase aqueuse.

La mise en contact de la phase aqueuse avec le solvant organique d'extraction du HMF peut être réalisée dans des appareillages classiques d'extraction liquide/liquide, en particulier dans des colonnes d'échange telles que des

colonnes pulsées.

Pour mettre en oeuvre le procédé de l'invention, on choisit les conditions de la réaction, c'est-à-dire les concentrations en hexose(s) et en catalyseur de la phase aqueuse, le temps de contact, la température et la pression de façon à obtenir une sélectivité élevée, c'est-à-dire un rapport

$$\frac{\text{moles de HMF formées}}{\text{moles d'hexose transformées}}$$

maximal, en particulier une sélectivité d'au moins 0,9.

En effet, si l'on se reporte au schéma réactionnel décrit ci-dessus qui fait intervenir la formation de produits intermédiaires et de sous-produits de dégradation du HMF, on peut définir les paramètres suivants de conduite de la réaction :

$$\text{- Sélectivité S} = \frac{\text{moles de HMF formées}}{\text{moles d'hexose transformées}}$$

$$\text{- Taux de transformation T} = \frac{\text{moles d'hexose transformées}}{\text{moles d'hexose initiales}}$$

$$\text{- Rendement de synthèse R} = \text{TxS} = \frac{\text{moles de HMF formées}}{\text{moles d'hexose initiales}}$$

Les conditions réactionnelles permettent d'agir sur ces paramètres. Ainsi, lorsque l'on augmente la température et/ou l'acidité, c'est-à-dire la quantité de catalyseur ou le rapport Si/Al, on accélère la transformation des hexoses, donc T augmente pour un temps donné, mais on favorise également les réactions de dégradation et de polymérisation si bien que S et R diminuent.

Selon l'invention, on vise une sélectivité S élevée plutôt qu'un taux de transformation T élevé.

Pour privilégier la sélectivité, il convient d'agir sur la réaction de transformation des produits intermédiaires en HMF pour éviter la transformation du HMF en polymère et la formation des sous-produits (acide lévulinique et acide formique).

Ceci peut être obtenu en extrayant au fur et à mesure le HMF produit dans un solvant organique. De plus, comme le HMF formé se trouve dans une solution peu acide ayant par exemple un pH supérieur à 3, donc peu propice à sa dégradation, on diminue les réactions secondaires de dégradation du HMF qui sont acido-catalysées.

Grâce au choix du catalyseur solide de l'invention, on peut toutefois réaliser la réaction à des températures relativement élevées, par exemple 100 à 200°C, pour obtenir néanmoins un taux de transformation des hexoses relativement élevé, restant compatible avec la sélectivité élevée recherchée.

La concentration en hexoses de la solution aqueuse et la concentration en catalyseur sont choisies en fonction des hexoses et du catalyseur utilisés pour obtenir la sélectivité élevée.

En effet, si l'acidité est élevée, c'est-à-dire si le rapport masse de catalyseur/masse de phase aqueuse est élevé, le taux de transformation des hexoses en produit intermédiaire sera élevé mais le taux de dégradation du HMF et le taux de polymérisation du produit intermédiaire risquent d'être aussi plus élevés. C'est pourquoi, selon l'invention, on privilégie la sélectivité au détriment du taux de transformation et on utilise un catalyseur solide capable d'adsorber sélectivement les produits de dégradation du HMF sans adsorber le HMF.

Généralement, la concentration en hexoses de la phase aqueuse est de 50 à 700 g/l. Selon les cas, la quantité de catalyseur est à définir de façon optimale en fonction de la vitesse de transformation du fructose et de la concentration des différents composés à l'intérieur de la phase aqueuse.

Selon un mode de réalisation avantageux du procédé de l'invention, on met en contact à co-courant ou à contre-courant dans plusieurs réacteurs d'extraction liquide/liquide :

- la phase aqueuse comprenant au moins un hexose ou un oside qui par hydrolyse donne au moins un hexose,
- le catalyseur solide acide constitué par un tectosilicate ou une argile sous forme protonique, et
- un solvant organique immiscible avec la phase aqueuse et capable de dissoudre l'hydroxyméthyl-5 furfural formé,

de façon à extraire en continu dans le solvant organique l'hydroxyméthyl-5 furfural formé en phase aqueuse, et on règle les quantités d'hexose, de catalyseur, de phase aqueuse et de solvant organique mis en courant, le débit de la phase aqueuse, le débit du solvant organique, la température et la pression de façon telle que, dans chaque réacteur d'extraction, la sélectivité, c'est-à-dire le rapport molaire HMF formé/hexose transformé soit supérieur à 0,9, de préférence supérieur à 0,95.

Dans ce cas, le rapport du débit du solvant organique au débit de la phase aqueuse est avantageusement de 1 à 100, de préférence de 1 à 10.

En effet, étant donné que le coefficient de partage, c'est-à-dire le rapport des concentrations à l'équilibre du HMF dans la phase organique et dans la phase aqueuse est voisin de 1, il est avantageux d'avoir un débit élevé de solvant organique par rapport au débit de la phase aqueuse pour extraire davantage de HMF.

Lorsque l'on utilise ainsi plusieurs réacteurs d'extraction, on peut faire circuler la phase aqueuse en série dans les réacteurs successifs pour transformer pratiquement toutes les molécules d'hexose de la phase aqueuse, et faire circuler le solvant organique en parallèle dans ces réacteurs pour améliorer l'extraction du HMF dans le solvant.

D'autres caractéristiques et avantages de l'invention apparaîtront à la lecture de la description qui suit donnée bien entendu à titre illustratif et non limitatif, se référant à la figure unique annexée.

Cette figure représente de façon schématique une installation à quatre colonnes d'extraction $C_1$, $C_2$, $C_3$ et $C_4$ qui peuvent être des colonnes pulsées, pour mettre en oeuvre le procédé de l'invention.

On introduit dans la première colonne $C_1$ par la conduite 1 une solution aqueuse d'hexose contenant en suspension un catalyseur solide constitué par une zéolithe sous forme $H^+$, chauffée à une température de 100 à 165°C, et on la met en contact à contre-courant avec un solvant organique tel que la méthylisobutylcétone qui est introduit par la conduite 3.

On récupère à la sortie de cette colonne :

- dans la conduite 5, une solution aqueuse appauvrie en hexose, ne contenant pratiquement pas de HMF, et
- dans la conduite 7, le solvant organique contenant le HMF produit dans la colonne C1.

La solution aqueuse est alors introduite dans la colonne $C_2$ où elle est mise en contact à contre-courant avec du solvant organique frais introduit par la conduite 9.

A la sortie de $C_2$, on récupère par la conduite 11 la solution aqueuse encore appauvrie en hexose et par la conduite 13 dans la conduite 7 le solvant organique contenant le HMF produit.

La solution aqueuse parcourt alors en série les colonnes $C_3$ et $C_4$ (conduites 11 et 15) où elle est mise en contact avec du solvant organique frais introduit par les conduites 17 et 21.

A la sortie de la dernière colonne, on récupère par la conduite 23 une solution aqueuse ne contenant pratiquement plus d'hexose et par la conduite 25 dans la conduite 7 du solvant organique contenant tout le HMF produit.

Le HMF est alors séparé du solvant par évaporation de ce dernier et récupéré sous forme concentrée. Après condensation du solvant évaporé, celui-ci est recyclé dans les conduites 3, 9, 17 et 21.

A titre d'exemple, on peut utiliser cette installation pour produire du HMF à partir d'une solution aqueuse de fructose contenant 1,2 mol/l de fructose et 30g/l de zéolithe circulant dans l'installation à un débit de 1 $m^3/h$ à contre-courant de méthylisobutylcétone introduit dans chaque colonne à un débit de 6 $m^3/h$ et en chauffant toutes les colonnes à une température de 160°C, afin d'obtenir une sélectivité d'au moins 0,9.

Les exemples 1 à 15, 17 et 18 qui suivent, illustrent l'emploi de catalyseurs solides conformes à l'invention. L'exemple 16 est donné à titre comparatif pour illustrer la synthèse du HMF sans catalyseur.

## EXEMPLE 1

Cet exemple concerne la production de HMF à partir de fructose en utilisant une zéolithe Y sous forme $H^+$ ayant un rapport Si/Al égal à 2,5.

On introduit dans un autoclave de 100 ml 50 ml d'eau, 2 g de fructose et 0,5 g de la zéolithe HY. Après agitation pendant quatre heures à 145°C, on prélève du liquide que l'on analyse par chromatographie liquide Haute Pression (HPLC) en utilisant la réfractométrie et la spectrométrie ultraviolette comme moyens de détection pour déterminer la teneur en HMF et en fructose de la solution aqueuse.

On trouve ainsi que la quantité de HMF formé représente 10 % en mole de la quantité de fructose de départ.

## EXEMPLE 2

Dans cet exemple, on suit le même mode opératoire que dans l'exemple 1 sauf que l'on utilise dans ce cas une zéolithe Y sous forme protonique ayant un rapport Si/Al égal à 10.

Dans ces conditions, la quantité de HMF formé est de 21 %.

## EXEMPLE 3

Dans cet exemple, on suit le même mode opératoire que dans l'exemple 1, mais on utilise une zéolithe HY dont le rapport Si/Al est égal à 20.

Dans ces conditions, la quantité de HMF formé est de 16 %.

## EXEMPLE 4

Dans cet exemple, on utilise le même mode opératoire que dans l'exemple 1 mais on utilise comme catalyseur de la montmorillonite sous forme protonique.

Dans ces conditions, la quantité de HMF formé est de 32 %.

Dans les quatre exemples, les quantités d'acide lévulinique, d'acide formique et de polymères insolubles après

réaction sont faibles.

EXEMPLE 5

Dans cet exemple, on utilise une zéolithe sous forme $H^+$ ayant un rapport Si/Al égal à 17, pour produire du HMF à partir de fructose.

On introduit dans un autoclave de 300 ml,

- 35 ml d'eau,
- 3,5 g de fructose,
- 1 g de zéolithe β, et
- 175 ml de méthylisobutylcétone.

On soumet l'ensemble à une agitation (700 tours/min), à 165°C, pendant 30 ou 60 min. Après décantation des deux phases, on dose le fructose et le HMF dans la phase aqueuse par HPLC (colonne SARACEPS CAR.H.) en réalisant la détection par réfractométrie, et on dose le HMF dans la phase organique par HPCL (colonne POLYMERS LABORATORIES PLRP.S) en réalisant la détection par spectrométrie UV.

On détermine à partir des valeurs trouvées lors de ces dosages :

- le taux de conversion (en %) qui correspond au rapport
$$\frac{\text{moles d'hexose transormées}}{\text{moles d'hexose initiales}},$$
- la sélectivité (en %) qui correspond au rapport
$$\frac{\text{moles de HMF formées}}{\text{moles d'hexose transformées}}$$
et
- le rendement (en %) qui correspond au rapport
$$\frac{\text{moles de HMF formées}}{\text{moles d'hexose initiales}}$$

Les résultats obtenus après 30 et 60 minutes de mise en contact sous agitation sont donnés dans le tableau annexé.

EXEMPLES 6 à 15

On suit le même mode opératoire que dans l'exemple 5 pour produire du HMF à partir de fructose, mais en utilisant 1 g des catalyseurs mentionnés dans le tableau au lieu de 1 g de zéolithe β.

Les résultats obtenus sont donnés également dans le tableau annexé.

EXEMPLE 16

Dans cet exemple, on suit le même mode opératoire que dans l'exemple 5 pour produire du HMF à partir de fructose, mais on n'utilise pas de catalyseur solide.

Les résultats obtenus sont donnés également dans le tableau.

Au vu des résultats du tableau, on constate que l'on peut obtenir des sélectivités pouvant atteindre plus de 90 % avec les tectosilicates sous forme $H^+$ de l'invention.

EXEMPLE 17

Dans cet exemple, on utilise la mordénite de l'exemple 13 pour produire du HMF à partir de fructose et on suit le même mode opératoire que dans l'exemple 5 sauf que l'on utilise 7 g de fructose.

Les résultats obtenus sont les suivants :

| | Après 30 min | Après 60 min | |
|---|---|---|---|
| Taux de conversion | 33 % | 55 % | |
| Sélectivité | 97 % | 92 % | |
| Rendement | 32 % | 51 % | |

EXEMPLE 18

Dans cet exemple, on suit le même mode opératoire que dans l'exemple 17, mais on opère dans un réacteur de 2 l, en utilisant

- 280 ml d'eau
- 56 g de fructose
- 1,2 l de méthylisobutylcétone et
- 8 g de mordénite H, Si/Al = 10.

On obtient les résultats suivants après 30 min d'agitation

Taux de conversion 57 %
Sélectivité : 97 %
Rendement : 56 %.

TABLEAU

| Ex | Catalyseurs | Si/Al | Temps (en min) | Conversion (%) | Sélectivité (%) | Rendement (%) |
|---|---|---|---|---|---|---|
| 5 | zéolithe β | 17 | 30<br>60 | 74<br>85 | 43<br>40 | 32<br>34 |
| 6 | zéolithe ZSM.5 | 25 | 30<br>60 | 82<br>90 | 37<br>39 | 30<br>35 |
| 7 | argile montmorillonite K10 | | 30<br>60 | 37<br>74 | 58<br>44 | 21<br>32 |
| 8 | zéolithe USHY | 2,5 | 30<br>60 | 41<br>89 | 30<br>23 | 12<br>20 |
| 9 | zéolithe HY | 10 | 30<br>60 | 85<br>89 | 52<br>46 | 44<br>41 |
| 10 | " | 15 | 30<br>60 | 57<br>75 | 54<br>52 | 31<br>39 |
| 11 | " | 20 | 60 | 45 | 52 | 23 |

TABLEAU (suite)

| Ex | Catalyseurs | Si/Al | Temps (en min) | Conversion (%) | Sélectivité (%) | Rendement (%) |
|---|---|---|---|---|---|---|
| 12 | zéolithe mordénite H | 6,9 | 30<br>60 | 43<br>55 | 97<br>98 | 41<br>54 |
| 13 | zéolithe mordénite H | 10 | 30<br>60 | 56<br>68 | 97<br>95 | 54<br>64 |
| 14 | " | 50 | 30<br>60 | 58<br>62 | 66<br>68 | 38<br>42 |
| 15 | zéolithe YNa | 2,5 | 30 | 43 | 41 | 17 |
| 16 | sans catalyseur | - | 30 | 15 | 40 | 6 |

## Revendications

1. Procédé de préparation d'hydroxyméthyl-5 furfural (HMF) par mise en contact d'un milieu liquide contenant au moins un nexose ou un oside qui par hydrolyse donne au moins un hexose, avec un catalyseur solide acide, à une température supérieure à 50°C, caractérisé en ce que le catalyseur acide est un tecto silicate ou une argile non fonctionnalisé par des groupes acides, sous forme protonique, et en ce que le milieu contenant l'hexose ou l'oside est un milieu aqueux.

2. Procédé selon la revendication 1, caractérisé en ce que le tectosilicate est une zéolithe.

3. Procédé selon la revendication 2, caractérisé en ce que la zéolithe a un rapport Si/Al allant de 2 à 100.

4. Procédé selon la revendication 1, caractérisé en ce que le catalyseur acide est une mordénite H.

5. Procédé selon la revendication 1, caractérisé en ce que l'on extrait l'hydroxyméthyl-5 furfural formé dans le milieu aqueux au moyen d'un solvant organique.

6. Procédé selon la revendication 5, caractérisé en ce que le solvant organique est un solvant sélectif du HMF, très peu soluble dans l'eau, dans lequel les hexoses et l'eau sont pratiquement insolubles et de point d'ébullition inférieur à celui du HMF.

7. Procédé selon la revendication 6, caractérisé en ce que le solvant organique est la méthylisobutylcétone.

8. Procédé selon l'une quelconque des revendications 1 à 7, caractérisé en ce que la température est de 100 à 200°C.

9. Procédé selon l'une quelconque des revendications 1 et 5, caractérisé en ce que l'on choisit les concentrations en hexose et en catalyseur acide de la phase aqueuse, la température et le temps de contact de façon à obtenir une quantité de HMF telle que la sélectivité, c'est-à-dire le rapport :

$$\frac{\text{moles de HMF formées}}{\text{moles d'hexose transformées}}$$

soit d'au moins 0,9.

10. Procédé selon l'une quelconque des revendications 1 à 9, caractérisé en ce que la transformation des hexoses en HMF s'effectue en présence du catalyseur acide solide, en milieu biphasique ou triphasique, à co-courant ou à contre-courant.

11. Procédé selon la revendication 10, caractérisé en ce que l'on met en contact à co-courant ou à contre-courant dans plusieurs réacteurs d'extraction liquide/liquide :

   - la phase aqueuse comprenant au moins une hexose ou un oside qui par hydrolyse donne au moins un hexose,
   - le catalyseur solide acide constitué par un tectosilicate ou une argile sous forme protonique, et
   - un solvant organique immiscible avec la phase aqueuse et capable de dissoudre l'hydroxyméthyl-5-furfural formé,

   de façon à extraire en continu dans le solvant organique l'hydroxyméthyl-5 furfural formé en phase aqueuse, et on règle les quantités d'hexose, de catalyseur, de phase aqueuse et de solvant organique mis en contact, le débit de la phase aqueuse, le débit du solvant organique, la température et la pression de façon telle que, dans chaque réacteur d'extraction, la sélectivité, c'est-à-dire le rapport molaire HMF formé/hexose transformé soit supérieur à 0,9 de préférence supérieure à 0,95.

## Patentansprüche

1. Verfahren zur Herstellung von 5-Hydroxymethylfurfural (HMF) durch Zusammenbringen eines flüssigen Mediums, das wenigstens eine Hexose oder eine Zuckerverbindung, welche durch Hydrolyse wenigstens eine Hexose liefert, enthält, mit einem festen sauren Katalysator bei einer Temperatur über 50°C, dadurch gekennzeichnet, daß der saure Katalysator ein Tectosilicat oder ein nicht mit sauren funktonellen Gruppen ausgestatteter Ton in Proton-Form ist und daß das die Hexose oder die Zuckerverbindung enthaltende Medium ein wäßriges Medium ist.

2. Verfahren nach Anspruch 1, dadurch gekennzeichnet, daß das Tectosilicat ein Zeolith ist.

3. Verfahren nach Anspruch 2, dadurch gekennzeichnet, daß der Zeolith ein Verhältnis von Si zu Al zwischen 2 und 100 hat.

4. Verfahren nach Anspruch 1, dadurch gekennzeichnet, daß der saure Katalysator ein H-Mordenit ist.

5. Verfahren nach Anspruch 1, dadurch gekennzeichnet, daß man das in dem wäßrigen Medium gebildete 5-Hydroxymethylfurfural mittels eines organischen Lösungsmittels extrahiert.

6. Verfahren nach Anspruch 5, dadurch gekennzeichnet, daß das organische Lösungsmittel ein für HMF selektives Lösungsmittel ist, das sehr wenig in Wasser löslich ist, in dem die Hexosen und das Wasser praktisch unlöslich sind und das einen niedrigeren Siedepunkt als HMF hat.

7. Verfahren nach Anspruch 6, dadurch gekennzeichnet, daß das organische Lösungsmittel Methylisobutylketon ist.

8. Verfahren nach einem der Ansprüche 1 bis 7, dadurch gekennzeichnet, daß die Temperatur zwischen 100 und 200°C liegt.

9. Verfahren nach einem der Ansprüche 1 und 5, dadurch gekennzeichnet, daß man die Konzentrationen der Hexose und des sauren Katalysators, die Temperatur und die Kontaktzeit derart wählt, daß man eine solche Menge HMF erhält, daß die Selektivität, d.h. das Verhältnis

$$\frac{\text{gebildete Mole HMF}}{\text{umgewandelte Mole Hexose}},$$

zumindest 0,9 beträgt.

10. Verfahren nach einem der Ansprüche 1 bis 9, dadurch gekennzeichnet, daß die Umwandlung der Hexosen in HMF in Gegenwart des festen sauren Katalysators im zweiphasigen oder dreiphasigen Medium im Gleichstrom oder im Gegenstrom erfolgt.

11. Verfahren nach Anspruch 10, dadurch gekennzeichnet, daß man in mehreren Flüssig-Flüssig-Extraktionsreaktoren im Gleichstrom oder im Gegenstrom in Kontakt bringt:

- die wäßrige Phase, die wenigstens eine Hexose oder eine Zuckerverbindung, welche durch Hydrolyse wenigstens eine Hexose liefert, umfaßt,
- den festen sauren Katalysator, der aus einem Tectosilicat oder aus einem Ton in Proton-Form besteht, und
- ein organisches Lösungsmittel, das mit der wäßrigen Phase nicht mischbar und imstande ist, das gebildete 5-Hydroxymethyl-furfural zu lösen,

um das in wäßriger Phase gebildete 5-Hydroxymethyl-furfural kontinuierlich in das organische Lösungsmittel zu extrahieren, und daß man die Mengen der Hexose, des Katalysators, der wäßrigen Phase und des in Kontakt gebrachten organischen Lösungsmittels, den Durchsatz der wäßrigen Phase, den Durchsatz des organischen Lösungsmittels, die Temperatur und den Druck derart reguliert, daß in jedem Extraktionsreaktor die Selektivität, d.h. das Molverhältnis von gebildetem HMF zu umgewandelter Hexose, über 0,9, vorzugsweise über 0,95 liegt.

**Claims**

1. Process for the preparation of 5-hydroxymethyl furfural (HMF) by contacting a liquid medium containing at least one hexose or a glycoside, which, by hydrolysis, gives at least one hexose, with a solid acid catalyst, at a temperature exceeding 50°C, characterized in that the acid catalyst is a tectosilicate or a clay not functionalized by acid groups, in protonic form, and in that the medium containing the hexose or glycoside is an aqueous medium.

2. Process according to claim 1, characterized in that the tectosilicate is a zeolite.

3. Process according to claim 2, characterized in that the zeolite has a Si/Al ratio from 2 to 100.

4. Process according to claim 1, characterized in that the acid catalyst is a mordenite H.

5. Process according to claim 1, characterized in that the 5-hydroxymethyl furfural formed in the aqueous medium is extracted by means of an organic solvent.

6. Process according to claim 5, characterized in that the organic solvent is a selective solvent of HMF, which is very slightly soluble in water, in which the hexoses and water are substantially insoluble and having a boiling point below that of HMF.

7. Process according to claim 6, characterized in that the organic solvent is methyl isobutyl ketone.

8. Process according to any one of the claims 1 to 7, characterized in that the temperature is 100 to 200°C.

9. Process according to either of the claims 1 and 5, characterized in that a choice is made of the hexose and acid catalyst concentrations of the aqueous phase, the temperature and the contact time in such a way as to obtain a HMF quantity such that the selectivity, i.e. the ratio of HMF moles formed to the hexose moles transformed is at least 0.9.

10. Process according to any one of the claims 1 to 9, characterized in that the transformation of hexoses into HMF takes place in the presence of the solid acid catalyst in a two or three-phase medium in counter-current or cocurrent manner.

11. Process according to claim 10, characterized in that cocurrent of countercurrent contacting takes place in several liquid/liquid extraction reactors of the aqueous phase incorporating at least one hexose or a glycoside which, by hydrolysis, gives at least one hexose, the solid acid catalyst constituted by a tectosilicate or a clay in protonic form and an organic solvent immiscible with the aqueous phase and able to dissolve the 5-hydroxymethyl furfural formed, so as to continuously extract in the organic solvent the 5-hydroxymethyl furfural formed in the aqueous phase and regulation takes place of the quantities of hexose, catalyst, aqueous phase and organic solvent made to flow, the flow rate of the aqueous phase, the flow rate of the organic solvent, the temperature and the pressure in such a way that, in each extraction reactor, the selectivity, i.e. the molar ratio of the HMF formed to the hexose transformed exceeds 0.9 and preferably exceeds 0.95.

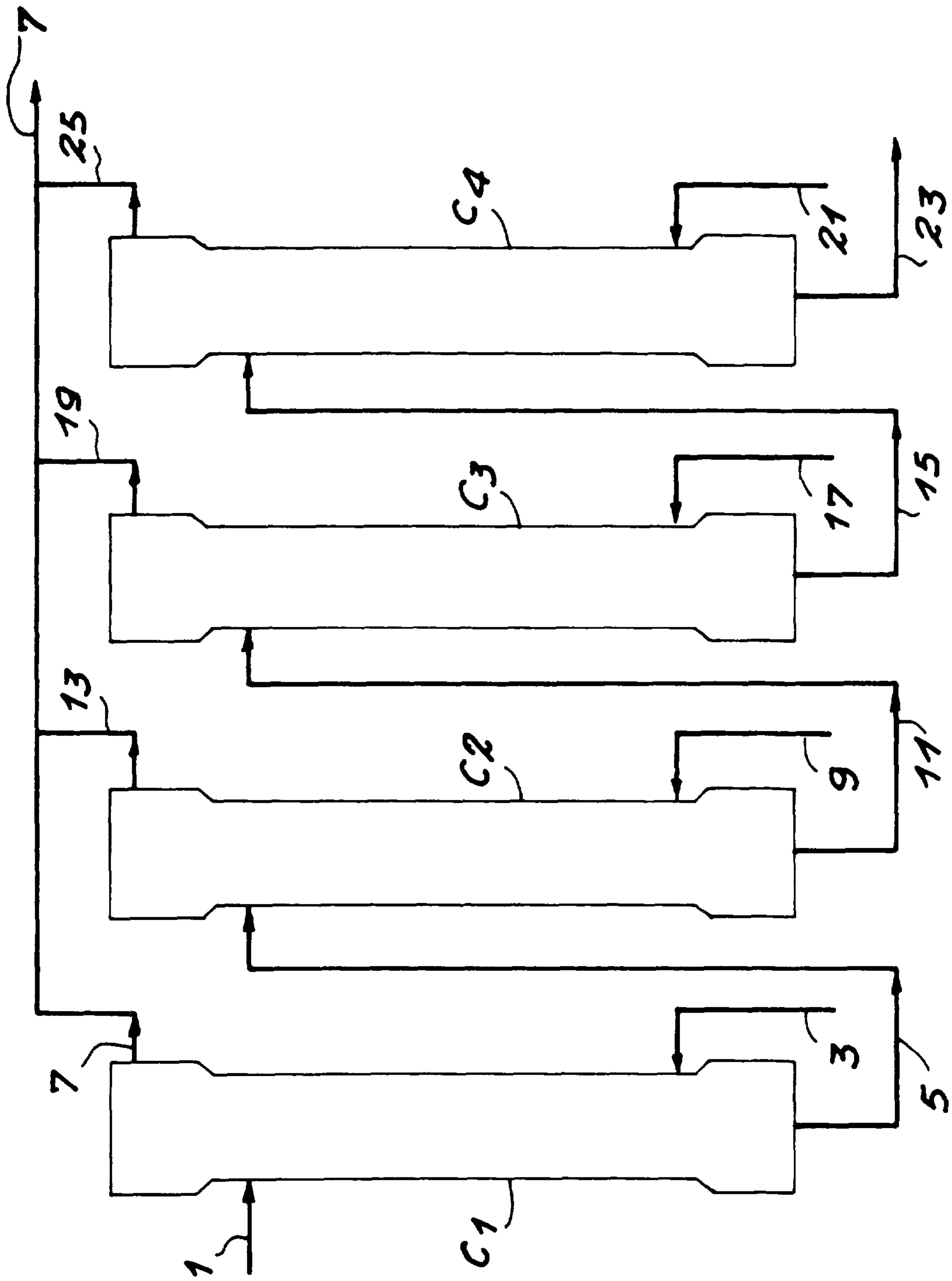
C1
C2
C3
C4
1
3
5
7
9
11
13
15
17
19
21
23
25